Europäisches Patentamt

European Patent Office

Office européen des brevets

(11) Veröffentlichungsnummer: **0 015 916**
**B1**

## EUROPÄISCHE PATENTSCHRIFT

(45) Veröffentlichungstag der Patentschrift: 21.04.82

(51) Int. Cl.³: **A 61 M 16/00**

(21) Anmeldenummer: **79900273.8**

(22) Anmeldetag: **15.03.79**

(86) Internationale Anmeldenummer: **PCT/CH 79/00041**

(87) Internationale Veröffentlichungsnummer: **WO 79/00788 (18.10.79** Gazette 79/21)

(54) **VORRICHTUNG ZUM KÜNSTLICHEN BEATMEN VON PATIENTEN.**

(30) Priorität: **17.03.78 CH 2973/78**

(43) Veröffentlichungstag der Anmeldung:
**01.10.80 Patentblatt 80/20**

(45) Bekanntmachung des Hinweises auf die Patenterteilung:
**21.04.82 Patentblatt 82/16**

(84) Benannte Vertragsstaaten:
**CH DE FR GB LU SE**

(56) Entgegenhaltungen:
**BE-A-496 982**
**DE-A-2 115 715**
**US-A-3 139 088**
**US-A-3 570 494**
**US-A-3 730 179**
**US-A-3 905 361**
**US-A-4 062 357**
**US-A-4 064 882**

(73) Patentinhaber: **SOCIETE TECHNIQUE POUR L'INDUSTRIE NOUVELLE S.A., 2, rue du Jura, CH-1800 Vevey (CH)**

(72) Erfinder: **REIST-KÜNDIG, Françoise, in der Müseren 14, CH-8122 Binz (CH)**
Erfinder: **VIGNOLA, Werner, c/o Gartmann Lütsch-Vorderberg, CH-8887 Mels (CH)**

(74) Vertreter: **Isler, Fritz et al, c/o Patentanwaltsbureau ISLER & SCHMID Walchestrasse 23, CH-8006 Zürich (CH)**

Vorrichtung zum künstlichen Beatmen von Patienten

Die vorliegende Erfindung betrifft eine Vorrichtung zum künstlichen Beatmen von Patienten mit einem Mundverschluß, gebildet aus einer Platte und einem diese durchsetzenden Rohrstück zum Einlegen zwischen das Zahnfleisch des Unter- und Oberkiefers einerseits und die Lippen und Wangen andererseits, und mit einem Nasenverschluß.

Aus der Literatur über Notfallmedizin ist es allgemein bekanntgeworden, daß der direkten Insufflationsbeatmung eine enorme Bedeutung zukommt, weil damit eine wirksame und auf ihre Wirksamkeit ununterbrochen kontrollierbare Beatmung augenblicklich beginnen kann und weil bekanntlich bei der respiratorischen Wiederbelebung mindestens Sekunden über den Erfolg entscheidend sein können. Daher wird es als unerläßlich betrachtet, daß jeder Helfer die direkte Insufflationsbeatmung ohne Verwendung eines Behelfes beherrscht. In Kursen über lebensrettende Sofortmaßnahmen wird diese auch fleißig an Beatmungsphantomen geübt. Es hat sich jedoch gezeigt, daß bei verletzten Personen, die blutverschmiert und schmutzig sind, nur gerade die zum Nothelferdienst verpflichteten Helfer, wie berufliches Sanitätspersonal, Polizisten, Feuerwehrleute, usw., diese unmittelbar lebensrettende Maßnahme wirklich durchführen, wogegen Laienhelfer oft schon wegen psychologischer Bedenken gegen den direkten Kontakt mit dem Patienten keine Atemspende durchführen und versagen.

Aus diesem Grunde wurden auch schon Mittel vorgeschlagen, die in Form einer einfachen Maske bis zu aufwendigen Einrichtungen auf dem Markt erhältlich sind, um die direkte körperliche Berührung zwischen Nothelfer und Patient zu vermeiden. Zudem gibt es, obwohl allgemein direkte Insufflationsbeatmung empfohlen wird, Fälle, wie beispielsweise Vergiftungs- und akute Infektionsgefahr, wo der hierbei notwendige Direktkontakt wegen der Selbstgefährdung des Helfers vermieden werden muß. An die zulässigen Beatmungsbehelfe werden jedoch vielfältige Bedingungen geknüpft. Sie müssen ohne Zeitverlust und ohne Schwierigkeiten die direkte Insufflationsbeatmung mit der Ausatmungsluft des Helfers ermöglichen. Zweckentsprechende Beatmungsbehelfe müssen eine überall wirksame Beatmung ermöglichen. Ihre Verwendung darf keinesfalls, auch bei Verwendung durch ungeübte Helfer, zu irgendwelcher Gefährdung des Patienten führen.

Es wurden bereits schon Masken vorgeschlagen, die auf Mund und Nase des Patienten aufgesetzt werden und mit einem Rohrstück versehen sind. Solche Masken verführen jedoch den ungeschulten Nothelfer dazu, den Unterkiefer nach unten und hinten zu drücken und damit die Luftwege in Höhe der hinteren Rachenwand durch die zurückfallende Zunge zu verschließen. Die Beatmung kann zusätzlich erschwert werden, wenn nicht sorgfältig auf die Lagerung des Kopfes zur Streckung der Atemwege im Rachen-Hals-Gebiet geachtet wird. Es wird häufig beobachtet, daß ungeschulte Nothelfer bei Manipulationen zum Dichthalten einer Maske den Kopf nicht mehr korrekt überstreckt halten, wodurch eine Beatmung unmöglich wird. Besonders bei aufgepreßten oder aufgebundenen Masken besteht dauernd Aspirationsgefahr mit nachfolgendem Atemstillstand. Todesfälle infolge dieses Zusammenhangs sind bekanntgeworden.

In der US-A-3 139 088 ist unter anderem ein Ausführungsbeispiel erläutert, bei dem eine vorgeformte Dichtungsplatte mit innerer konkaver und äußerer konvexer Oberfläche auf einem diese Platte zentral durchstoßenden Rohr gleitbar geführt ist. Das Rohr ist mit einem Wulst zur steckbaren Verbindung mit einem Endotrachealtubus versehen und die Platte hat die Aufgabe, in Zusammenwirkung mit den Lippen eine Dichtung für die Mundöffnung zu bilden. Diese Platte soll dementsprechend in den dentolabialen Raum eingesetzt werden. Infolge der Dicke dieser Platte werden jedoch die Lippen nach außen hin von ihrer Auflage auf den Zähnen abgehoben, so daß beispielsweise bei einem bewußtlosen Patienten der Dichtungseffekt fraglich ist.

Es ist eine Aufgabe der Erfindung, eine eingangs beschriebene Vorrichtung zu schaffen, die von jedem Helfer, ob geübt oder ungeübt, als Beatmungsbehelf zur Atemspende eingesetzt werden kann und mit der keine Verletzung des Patienten befürchtet werden muß.

Zusätzlich soll auch eine klinische Anwendung ermöglicht werden, insbesondere

(1) für die Anwendung von Beatmung unter kontinuierlich positivem Luftwegdruck, in der Fachsprache CPAP (Continuous Positive Airway Pressure) genannt, ohne eine endotracheale Intubation beim spontan atmenden Patienten, und
(2) für die Durchführung einer wechselnden positiven Überdruckbeatmung, in der Fachsprache IPPB (Intermittent Positive Pressure Breathing) genannt, während der respiratorischen Physiotherapie.

Erfindungsgemäß wird dies mit einer Vorrichtung, die in den Patentansprüchen beschrieben ist, erreicht.

Ausführungsbeispiele der Erfindung werden nachfolgend an Hand der Zeichnung näher erläutert. Dabei zeigt:

Fig. 1 einen Grundriß eines erfindungsgemäßen Mundverschlusses,

Fig. 2 einen Schnitt durch den Mundverschluß nach Fig. 1 gemäß der Schnittlinie 2-2,

Fig. 3 einen Seitenriß eines menschlichen Kopfes, zum Teil geschnitten, mit eingesetztem Mundverschluß nach der Erfindung.

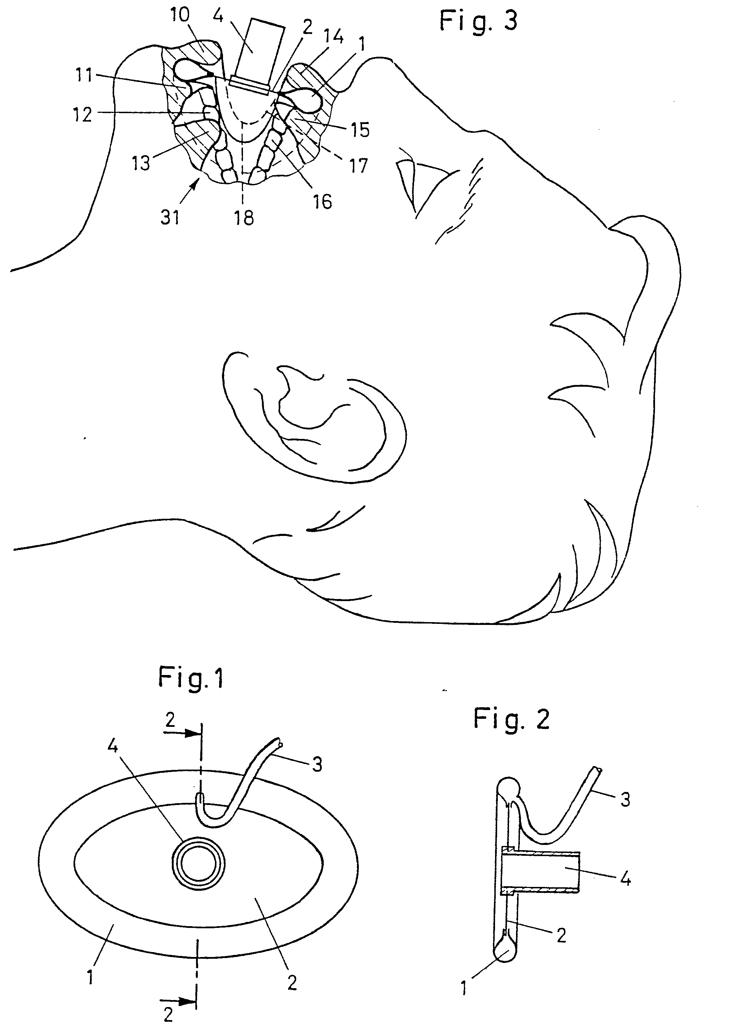

Der Mundverschluß gemäß Fig. 1 und 2 besteht aus einem aufblasbaren Pneu 1 mit einem Luftzufuhrschlauch 3 und aus einer verformbaren Platte 2 und einem diese Platte 2 durchsetzenden Rohrstück 4. Die Platte 2 kann beispielsweise aus Blech gefertigt sein, das als Schutz vor Korrosion oder zur Befestigung des Pneus beidseitig mit einer Kunststoff-Folie überdeckt sein kann.

Als Pneu 1 ist beispielsweise ein aufblasbarer Ring vorgesehen, der am Umfang der Platte 2 befestigt ist. Ein in den Pneu eindringender Schlauch 3 dient zum Aufblasen des Pneus. Im Zentrum der Platte 2 ist ein Rohrstück 4 eingesetzt, das die Platte 2 durchsetzt und gegenüber dieser abgedichtet ist.

An Stelle eines Luftpneus gemäß der gezeigten Ausführungsform könnte auch ein Wulst aus geschäumtem Kunststoff vorgesehen sein. Diese letztgenannte Ausführungsform würde sich besonders gut für Nothelfer eignen, da kein bestimmter Luftdruck im Pneu hergestellt und auch überwacht werden muß.

Die Grundform des Mundverschlusses ist, wie Fig. 1 darstellt, elliptisch mit einer kurzen Ellipsenachse, die — bei einem Schädel mit mittleren Massen — im dentilabialen Raum des Unter- und des Oberkiefers endet. Die lange Ellipsenachse ist dann derart gewählt, daß der Pneu hinter den Mundwinkeln die Zahnreihen überquert.

Wie Fig. 3 zeigt, liegt — bei einem Schnitt durch den Mundverschluß etwa bei der Schnittlinie 2-2 — der Pneu 1 im dentilabialen Raum des Unterkiefers zwischen der Unterlippe 10 und dem Zahnfleisch 11 mit der Zahnreihe 12 und im dentilabialen Raum des Oberkiefers zwischen der Oberlippe 14 und dem Zahnfleisch 15 mit der Zahnreihe 16. Die Zunge 13 ist lediglich angedeutet. Bei leicht geöffneten Lippen 10, 14 ergibt sich angenähert eine Mundöffnung nach der Linie 17. Dieser Schnitt zeigt deutlich, daß der Pneu 1 des Mundverschlusses hinter den Mundwinkel 18 zu liegen kommt und somit auch seitlich eine Abdichtung der Mundhöhle gegen die Außenluft bewirkt.

Bei der Erprobung der erfindungsgemäßen Vorrichtung sowohl durch ungeübte Nothelfer als auch bei der Anästhesie in einer Klinik zeigte sich der große Vorteil gegenüber allen heute bekannten Vorrichtungen.

In der klinischen Erprobung konnte der Nachweis erbracht werden, daß bei Anwendung von CPAP (kontinuierlicher positiver Luftwegdruck) am spontan atmenden Patienten eine endotracheale Intubation nicht mehr notwendig ist, denn durch den Mundverschluß und den Nasenverschluß werden Atemwege und Lungen gesamthaft von der Außenwelt abgeschlossen und die Spontanatmung kann unter einem kontinuierlichen Überdruck von 0,005 bis 0,01 kg/cm² in gleicher Weise wie vorher über den Tubus sichergestellt werden. Die beschriebene Vorrichtung erlaubt, wie festgestellt, die Anwendung von CPAP. Aber auch eine Anwendung von positivem endexspiratorischem Druck (PEEP=Positive End-Exspiratory Pressure), was neben dem CPAP bei gewissen Formen von Atemnotsyndrom häufig als Therapie eingesetzt wird, ist möglich ohne Intubation. Beide, der kontinuierliche positive Atemwegdruck (CPAP) und der positive endexspiratorische Druck (PEEP) zeigen sich bekanntlich besonders wertvoll bei der Entwöhnung eines Patienten von einem Respirator. Bisher mußte bei dieser Entwöhnungsphase für einen wirkungsvollen Einsatz des endexspiratorischen Druckes (PEEP) bei spontanatmenden Patienten der endotracheale Tubus zur Durchführung der kontrollierten respiratorischen Beatmung oft noch während längerer Zeit belassen werden. Der erfindungsgemäße Mundverschluß, zusammen mit einem Nasenverschluß, erlaubt dagegen schon früh eine Extubation, weil die respiratorischen Maßnahmen jederzeit durch einfaches Einsetzen des Mund- und des Nasenverschlusses eingesetzt, abgebrochen oder wieder aufgenommen werden können, so daß der Patient die für die Lunge notwendige Dehn- und Säuberungsaktionen, wie Gähnen, Räuspern, usw. ausführen kann, was bei einer Intubation nicht der Fall ist.

Es ist somit klar ersichtlich, daß der Tubus bis zu einigen Tagen früher entfernt werden kann, was für die Prophylaxe der als äußerst gefährlich bekannten Trachealstenose in hohem Maße dienlich ist. Darüber hinaus können nun auch Patienten mit akutem Atemnotsyndrom des Erwachsenen (ARDS=Acute Respiratory Distress Syncrome) mit kontinuierlichem positivem Luftwegdruck (CPAP) behandelt werden, wobei die endotracheale Intubation vermieden werden kann.

Eine Behandlung unter kontinuierlichem positivem Atemwegdruck (CPAP) oder unter endexspiratorischem Druck (PEEP) ist bei vielen Störungen der Lungenfunktion angezeigt (Verletzung der Lunge, Schocklunge, nach Abdominaloperationen, Rippenserienfraktur, Aspiration von Magensaft oder ganz einfach bei bettlägerigen Patienten), die zu einem Verschluß der kleinen Luftwege führen können, so daß die nicht-ventilierten Alveolen dem Blut keinen Sauerstoff mehr abgeben und dadurch der Sauerstoffdruck im Blut absinkt.

Auch bei der Erprobung am Beatmungsbehelf bei der Insufflationsbeatmung hat sich dieser Mundverschluß als vorteilhaft erwiesen, indem das Rohrstück 4, das die Platte 2 des Mundverschlusses durchsetzt, einen Abstand zum Patienten schafft, und damit der Abscheu vor der Berührung eines bewußtlosen und/oder verschmutzten Menschen bedeutend vermindert wird. Bei dieser Anwendung des Mundverschlusses und bei manuellem Verschluß der Nasenlöcher war kein Leck bei der Beatmung feststellbar und damit kein Luftverlust aus der Mundhöhle. Wegen der logischen Tendenz des Nothelfers, die Mundplatte (mit der Hand des Helfers und den Lippen des Patienten fixiert) und das Rohrstück seinem Mund zur Insufflation entgegenzuführen, werden automatisch Kiefer und Kopf des Patienten in die für eine Beatmung unbedingt erforderliche gestreckte Haltung gebracht.

Selbstverständlich muß für diesen Fall der Pneu 1 nicht aufblasbar sein, sondern er kann vorzugsweise aus einem geschäumten Kunststoff bestehen.

Bei einem Pneu 1 aus elastischer Folie, aus künstlichem oder natürlichem Kautschuk, kann im Luftzufuhrschlauch 3 ein gleichartiger Ballon vorgesehen werden, wie der Ballon 42 mit Rückschlagventil 43, der beim Nasenverschluß dargestellt ist. Dieser Ballon gestattet die Überprüfung des Druckes im Pneu, wenn der jeweilige Verschluß nicht dauernd an Druckluft angeschlossen bleibt.

**Patentansprüche**

1. Vorrichtung zum künstlichen Beatmen von Patienten mit einem Mundverschluß, gebildet aus einer gekrümmten elastisch verformbaren Abdichtungsscheibe, die von einem Einblasrohr zentral durchsetzt ist, dadurch gekennzeichnet, daß die Scheibe zum dichtenden Einsetzen in den dentolabialen Raum des Patienten als blattförmig flache und elastisch verformbare Platte (2) mit beidseitig der Platte angeordnetem weichelastischem Randwulst (1) ausgebildet ist und daß das Einblasrohr (4) einseitig von der Plattenmitte vorstehend ausgebildet ist.

2. Vorrichtung nach Anspruch 1, dadurch gekennzeichnet, daß der Wulst (1) ein mit einem Gas aufblasbarer Pneu ist.

3. Vorrichtung nach Anspruch 1, dadurch gekennzeichnet, daß der Wulst (1) aus weichgeschäumtem Kunststoff besteht.

4. Vorrichtung nach Anspruch 1, dadurch gekennzeichnet, daß die Abmessungen der Platte derart sind, daß die kürzere Ellipsenachse zwischen 2 und 5 cm lang ist, um in den dentolabialen Räumen bei geschlossenem Mund zwischen Zahnfleisch und Lippen zu dichten, und die längere Ellipsenachse zwischen 5 und 10 cm lang ist, um zwischen Zahnfleisch und Wangen zu dichten.

5. Vorrichtung nach Anspruch 1, dadurch gekennzeichnet, daß der Wulst einen wenigstens angenähert kreisförmigen Querschnitt aufweist.

**Claims**

1. Device for artificial respiration of patients with a sealing member for the mouth, formed of a curved resiliently deformable sealing disc which is centrally penetrated by an inflation tube, characterized in that the disc to be inserted as a seal into the patient's dentolabial area is formed as a leaf-shaped flat and resiliently deformable plate (2) with a flexible surrounding bulge (1) disposed on either side of the plate and in that the inflation tube (4) is formed so as to project on one side of the plate centre.

2. Device according to claim 1, characterized in that the bulge (1) is a tube which can be inflated with gas.

3. Device according to claim 1, characterized in that the bulge (1) is made of soft foamed plastic.

4. Device according to claim 1, characterized in that the dimensions of the plate are such that the shorter elliptic axis is between 2 and 5 cm long in order to form a seal between the gum and lips in the dentolabial areas when the mouth is closed, and the longer elliptic axis is between 5 and 10 cm long in order to form a seal between the gum and cheeks.

5. Device according to claim 1, characterized in that the surround has a cross-section which is at least approximately circular.

**Revendications**

1. Dispositif pour la respiration artificielle de patients avec un obturateur buccal, formé par une plaque d'étanchéité curviligne, déformable élastiquement et traversée en son centre par un tube d'insufflation, caractérisé par le fait que la plaque, pour être mise en place de manière étanche dans la cavité dentolabiale du patient, est conformée en une plaquette (2) plate en forme de feuille et déformable élastiquement, avec un bourrelet périphérique (1) élastique et mou situé de part et d'autre de ladite plaquette, et par le fait que le tube d'insufflation (4) fait saillie d'un côté au-delà du centre de ladite plaquette.

2. Dispositif selon la revendication 1, caractérisé par le fait que le bourrelet (1) est un organe pneumatique pouvant être gonflé avec un gaz.

3. Dispositif selon la revendication 1, caractérisé par le fait que le bourrelet (1) consiste en une mousse tendre de matière plastique.

4. Dispositif selon la revendication 1, caractérisé par le fait que les dimensions de la plaquette sont telles que l'axe le plus court de l'ellipse mesure de 2 à 5 cm de long, pour assurer l'étanchéité dans les cavités dentolabiales entre les gencives et les lèvres lorsque la bouche est fermée, et que l'axe plus long de l'ellipse présente une longueur de 5 à 10 cm, pour assurer l'étanchéité entre les gencives et les joues.

5. Dispositif selon la revendication 1, caractérisé par le fait que le bourrelet présente une section au moins approximativement circulaire.

0 015 916

Fig. 3

Fig. 1

Fig. 2